# EUROPEAN PATENT APPLICATION

(11) **EP 3 831 305 A1**
(43) Date of publication of application: **09.06.2021**
(21) Application number: 19843366.6
(22) Date of filing: 14.06.2019
(51) Int. Cl.: A61B 6/00, A61B 6/03

(54) **DEVICE FOR SUPPORTING BONE MARROW READING ON BASIS OF IMAGE ANALYSIS**

(30) Priority: 02.08.2018 KR 20180090182
(71) Applicant: UIMD INC., Mapo-gu Seoul (KR)
(72) Inventor: LEE, Young Deuk, Seoul 04196 (KR)
(74) Representative: M. Zardi & Co S.A.
(86) International application number: PCT/KR2019/007194
(87) International publication number: WO 2020/027431

(57) **Abstract**

The present invention relates to a device for supporting bone marrow reading based on image analysis. According to the present invention, the device for supporting bone marrow reading based on image analysis comprises: an image acquisition unit for acquiring a bone marrow image of a subject for bone marrow reading by an imaging device; a discrimination coefficient unit for classifying the acquired bone marrow image into one class among a plurality of classes according to preset discrimination coefficients; and a result providing unit for providing the result read by the discrimination coefficient unit.

## Description

### [Technical Field]

The present invention relates to a device for supporting bone marrow reading, and more particularly, to a device for supporting bone marrow reading based on image analysis capable of efficiently and easily performing bone marrow reading by providing images used for bone marrow reading and classifying bone marrow cells into different classes for each type to provide discrimination coefficients.

### [Background Art]

Bone marrow examination is one of diagnostic testing methods performed by collecting bone marrow, which is a hematopoietic organ. Such bone marrow examination is performed not only for diagnosis of various blood diseases and follow-up tests after treatment, but also to determine whether various cancers have metastasis to the bone marrow, and to determine whether granulomatous diseases such as tuberculosis are invaded.

Diagnostic hematologists make a diagnosis by observing stained bone marrow smear samples and bone marrow biopsy tissue sections under a microscope and synthesizing the discrimination coefficients of bone marrow cells to read the bone marrow examination. In addition, if necessary, a comprehensive diagnosis should be made by observing several types of specially stained smear slides or immunostained biopsy tissue section slides. This task requires a lot of experience and knowledge, and is a task that is accompanied by severe eye fatigue because it is necessary to observe various parts of the slide at high magnification and an observation doctor has to spend a lot of time.

Also, if it is difficult to read the bone marrow, it is necessary to ask for opinions of other experts. However, currently, there is an inconvenience in that the bone marrow slides are packaged and sent by parcel post or courier service, the bone marrow slides are observed again under a microscope at a place which received the bone marrow slides, the results are sent to wires or paper, and the slides are returned. Due to the inconvenience and the demand of a lot of time, there is a problem in that information required on the day of the examination cannot be delivered to a clinical doctor treating a patient.

In the related art, in order to use a bone marrow image, a person has to take a microscopic picture, but since the image quality is poor and only cells in a desired part are imaged by the observer, this method alone cannot be used for diagnosis. In addition, a bone marrow reading education method is limited to use in practical education because there are only apprenticeship forms with many limitations that require microscopic observation, and even if there is an atlas, there are only a few typical cell type photographs. For the same reason, there is no quality control method to determine whether the bone marrow examination is properly read.

### [Disclosure]

### [Technical Problem]

The present invention is derived to solve the problems, and an object of the present invention is to provide a device for supporting bone marrow reading based on image analysis capable of providing all images used for bone marrow reading in sufficient numbers at a required magnification using an image analyzer, capturing 500 images of bone marrow cells or the number of images of bone marrow cells specified by a customer at high magnification to classify and provide the images into different classes for each cell type using an algorithm, and enhancing the efficiency and convenience of bone marrow reading by providing the images.

### [Technical Solution]

According to the present invention to solve the object, a device for supporting bone marrow reading based on image analysis includes: an image acquisition unit configured to acquire a bone marrow image of a subject for bone marrow reading by an imaging device; a discrimination coefficient unit configured to generate the acquired bone marrow image as a required image by image processing and software control and classify the acquired bone marrow image into one class among a plurality of classes included in discrimination coefficients by a preset algorithm; and a result providing unit configured to provide the required image and the result discriminated by the discrimination coefficient unit.

The required image may be a large particle having a size larger than that of other parts around a whole bone marrow image screen.

The required image may include the large particles and megakaryocytes existing in a zone of interest before and behind the large particles.

The result providing unit may provide both the required image and a discrimination coefficient corresponding to a class to which the required image belongs.

The present invention may further include a database unit in which the plurality of bone marrow images classified by class according to the preset discrimination coefficients are stored, wherein the result providing unit may provide both the data analyzed by the discrimination coefficient unit and the data stored in the database unit.

### [Advantageous Effects]

The device for supporting bone marrow reading based on image analysis according to the present invention having the configuration as described above is configured to classify and provide all the bone marrow images acquired by an imaging means such as CT into one class among different classes classified according to discrimination coefficients. Accordingly, the present invention has advantages of saving labor and time by performing bone marrow reading without microscopic observation, consulting the bone marrow reading with other experts or reading the bone marrow images remotely at a place such as a central reading center by reading the bone marrow images by discrimination coefficients reflected with an algorithm for discrimination of bone marrow cells, and providing educational data of a complete image configuration for bone marrow reading doctors.

### [Description of Drawings]

FIG. 1 is a configuration diagram for describing a configuration of a device for supporting bone marrow reading based on image analysis according to an embodiment of the present invention,
FIG. 2 is a flowchart sequentially illustrating a process of an embodiment of the present invention,
FIG. 3 is a flowchart sequentially illustrating a reading process of a discrimination coefficient unit applied to an embodiment of the present invention,
FIG. 4 is a diagram showing an example of an image acquired by an image acquisition unit applied to an embodiment of the present invention,
FIG. 5 is a diagram showing an enlarged photograph of an image around a large particle in FIG. 4, and
FIG. 6 is a diagram showing a photograph of an example of a result provided by a result providing unit applied to an embodiment of the present invention.

### [Modes of the Invention]

Hereinafter, embodiments of the present invention will be described in detail with reference to the accompanying drawings and the contents disclosed in the accompanying drawings, but the present invention is not limited or restricted to the embodiments.

Various modifications may be made to embodiments to be described below. Embodiments to be described below are not intended to be limited to aspects and should be understood to include all modifications, equivalents, and substitutes thereof.

Terminologies used herein are used only to describe specific embodiments, and are not intended to limit the embodiments. A singular form may include a plural form unless otherwise clearly meant in the context. Further, in the present application, it should be understood that the term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Meanwhile, in describing the present invention, detailed description of associated known function or constitutions will be omitted if it is determined that they unnecessarily make the gist of the present invention unclear. Terminologies used herein are a terminologies used to properly express embodiments of the present invention, which may vary according to a user, an operator's intention, or customs in the art to which the present invention pertains. Accordingly, definitions of the terminologies need to be described based on contents throughout this specification.

In describing the embodiments, a detailed description of related known technologies will be omitted if it is determined that they unnecessarily make the gist of the embodiments unclear.

### Device for supporting bone marrow reading based on image analysis

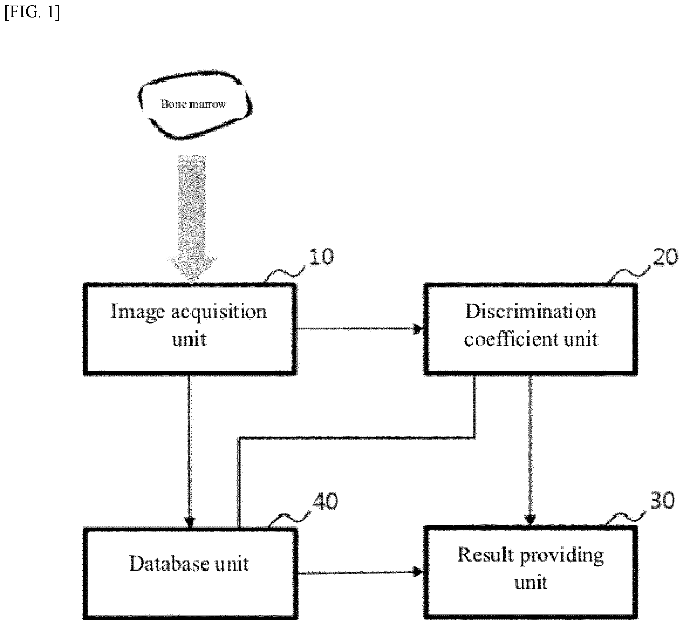

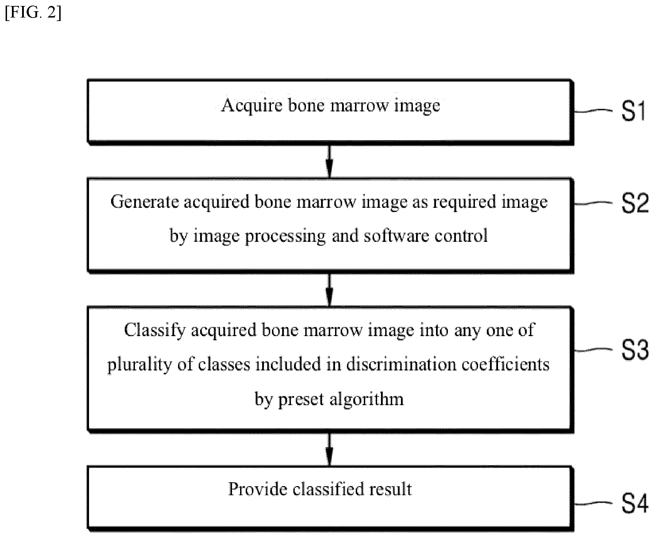

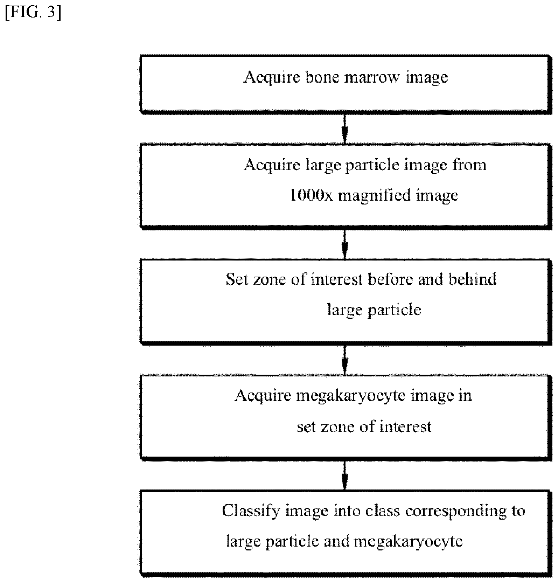

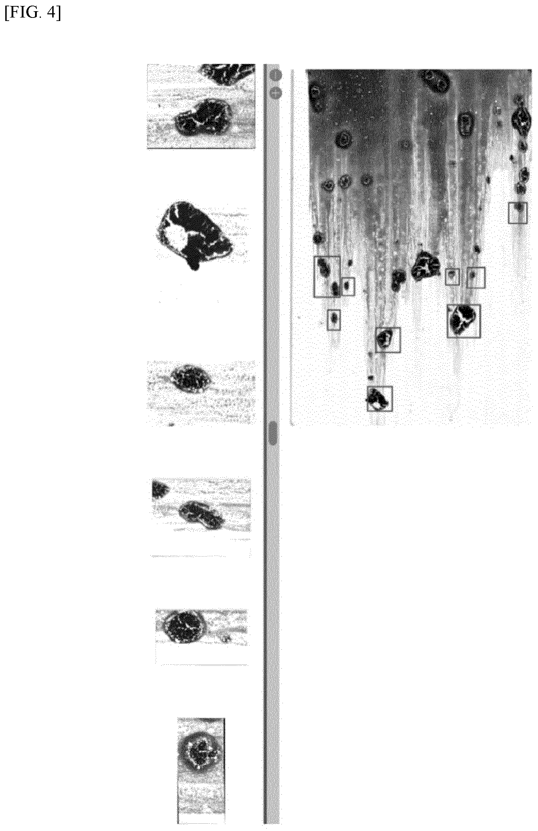

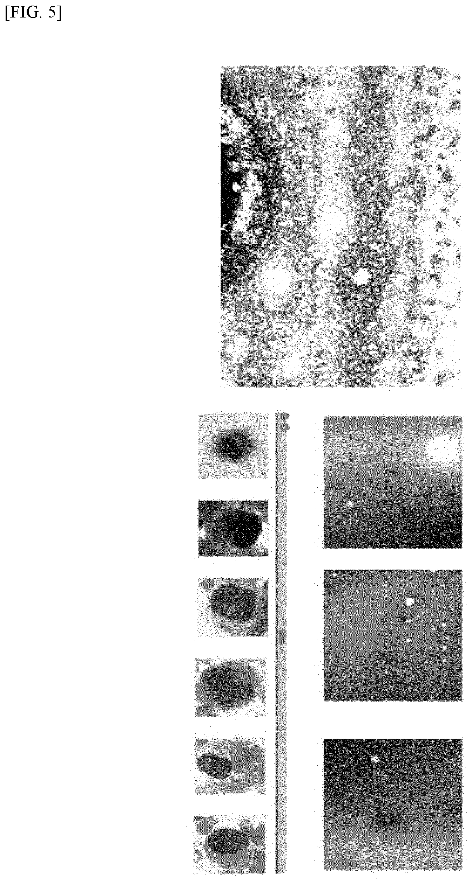

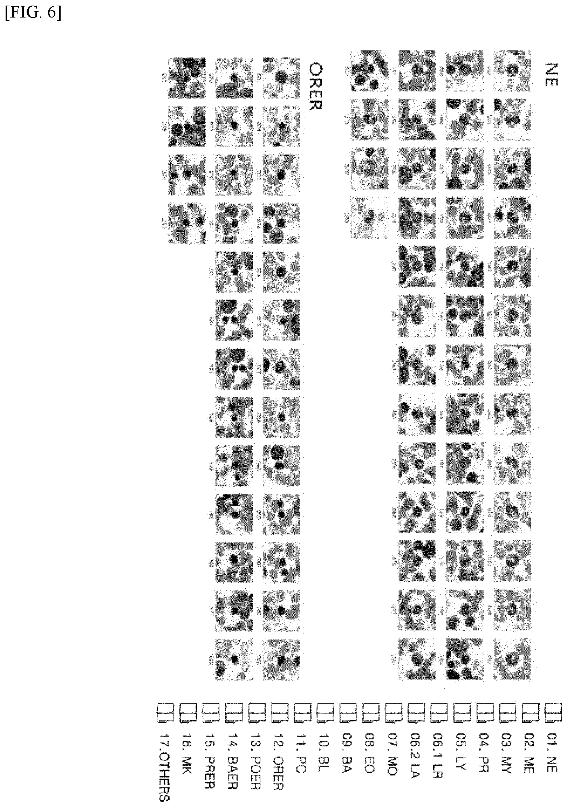

FIG. 1 is a configuration diagram for describing a configuration of a device for supporting bone marrow reading based on image analysis according to an embodiment of the present invention, FIG. 2 is a flowchart sequentially illustrating a process of an embodiment of the present invention, FIG. 3 is a flowchart sequentially illustrating a reading process of a discrimination coefficient unit 20 applied to an embodiment of the present invention, FIG. 4 is a diagram showing an example of an image acquired by an image acquisition unit 10 applied to an embodiment of the present invention, FIG. 5 is a diagram showing an enlarged photograph of an image around a large particle in FIG. 4, and FIG. 6 is a diagram showing a photograph of an example of a result provided by a result providing unit 30 applied to an embodiment of the present invention.

As illustrated in FIGS. 1 and 2, the device for supporting bone marrow reading based on image analysis according to an embodiment of the present invention includes an image acquisition unit 10, a discrimination coefficient unit 20, and a result providing unit 30.

The image acquisition unit 10 serves to acquire images of bone marrow cells as shown in FIGS. 4 and 5 using an imaging means such as CT (S1) so as to capture and provide all images used for bone marrow reading to a doctor.

The discrimination coefficient unit 20 generates the acquired bone marrow image as a required image by image processing and software control (S2) as shown in FIGS. 4 and 5, and classifies the acquired bone marrow image into any one of a plurality of classes included in discrimination coefficients by a preset algorithm (S3).

The required image is, for example, a large particle having a size larger than that of other parts around a whole bone marrow image screen, and in the embodiment, a required image that is a specified number of large particles is captured and provided.

In the embodiment, cells existing in a zone of interest before and behind the large particles are generated as a required image together with the large particles, and the generated required image is configured to be provided as many as a specified number. That is, for determining megakaryocytes, a sufficient size is defined before the particles to be captured and provided as many as the specified number, and the megakaryocytes are detected with software and the high-magnification image is captured and provided.

The discrimination coefficient of the bone marrow cell is a coefficient set by a cell classification algorithm using a high-magnification cell image acquired from a region where the cells are well-smeared, and the result providing unit 30 provides the discrimination coefficients together with the required images (S4).

For example, the result providing unit 30 may be configured to provide a specified number of particle images, zone-of-interest images, and high-magnification cell images when a slide on which bone marrow to be read is placed is a special stained smear slide other than basic bone marrow staining. In addition, the number of all provided images may be set by the consumer. Bone marrow tissue sections are provided as the entire image for general staining, immunostaining, and special staining, and are clicked by the customer and enlarged in a desired size to be observed.

Here, the discrimination coefficient may be set in various methods, but it is preferable to set the discrimination coefficient in consideration of characteristic factors of the bone marrow. For example, the discrimination coefficient is preferably set based on a region that best represents the findings of the bone marrow because peripheral blood contamination is least in adjacent regions of the bone marrow cells before and behind the particles having relatively large sizes.

Therefore, the discrimination coefficient unit 20 applied to the embodiment, as well illustrated in FIG. 3, classifies the bone marrow images into different classes based on discrimination coefficients set according to the size and shape of large particles having a larger size than other surrounding particles, and the size and shape of megakaryocytes in the zone of interest before and behind the large particles on the bone marrow image screen.

The result providing unit 30 serves to provide both the required image and the result discriminated by the discrimination coefficient unit 20, as well illustrated in FIG. 6.

The device for supporting bone marrow reading based on image analysis according to an embodiment of the present invention having the configuration as described above is configured to classify and provide all the bone marrow images acquired by an imaging means such as CT into one class among different classes classified according to discrimination coefficients. Accordingly, the present invention are has advantages of saving labor and time by performing bone marrow reading without microscopic observation, consulting the bone marrow reading with other experts or reading the bone marrow images remotely at a place such as a central reading center by reading the bone marrow images by discrimination coefficients reflected with an algorithm for discrimination of bone marrow cells, and providing educational data of a complete image configuration for bone marrow reading doctors.

The embodiment further includes a database unit 40 in which the plurality of bone marrow images classified by class according to preset discrimination coefficients are stored. The result providing unit 30 is configured to provide both the data analyzed by the discrimination coefficient unit 20 and the data stored in the database unit 40. Accordingly, since the user can compare and analyze the actually read bone marrow image and the databased bone marrow image, there is an effect of enabling more precise bone marrow reading.

Although various embodiments of the present invention have been described above, the present embodiment and the accompanying drawings are merely illustrative of some of the technical ideas included in the present invention. It will be apparent that modified examples and specific embodiments that can be easily inferred by those skilled in the art within the scope of the technical idea included in the specification and drawings of the present invention are included in the scope of the present invention.

### [Explanation of Reference Numerals and Symbols]

- 10:: Image acquisition unit
- 20:: Discrimination coefficient unit
- 30:: Result providing unit
- 40:: Database unit

## Claims

1. A device for supporting bone marrow reading based on image analysis comprising:
an image acquisition unit configured to acquire a bone marrow image of a subject for bone marrow reading by an imaging device;
a discrimination coefficient unit configured to generate the acquired bone marrow image as a required image by image processing and software control and classify the acquired bone marrow image into one class among a plurality of classes included in discrimination coefficients by a preset algorithm; and
a result providing unit configured to provide the required image and the result discriminated by the discrimination coefficient unit.

2. The device for supporting bone marrow reading based on image analysis of claim 1, wherein the required image is a large particle having a size larger than that of other parts around a whole bone marrow image screen.

3. The device for supporting bone marrow reading based on image analysis of claim 2, wherein the required image includes the large particles and megakaryocytes existing in a zone of interest before and behind the large particles.

4. The device for supporting bone marrow reading based on image analysis of claim 3, wherein the result providing unit provides both the required image and a discrimination coefficient corresponding to a class to which the required image belongs.

5. The device for supporting bone marrow reading based on image analysis of claim 1, further comprising:
a database unit in which the plurality of bone marrow images classified by class according to the preset discrimination coefficients are stored,
wherein the result providing unit provides both the data analyzed by the discrimination coefficient unit and the data stored in the database unit.
